# EUROPEAN PATENT APPLICATION

(11) **EP 4 574 999 A1**
(43) Date of publication of application: **25.06.2025**
(21) Application number: 23383369.8
(22) Date of filing: 22.12.2023
(51) Int. Cl.: C12Q 1/6886

(54) **IN VITRO METHOD FOR PREDICTING LONGEVITY IN PATIENTS SUFFERING FROM BREAST CANCER**

(71) Applicant: Reveal Genomics S.L., 08036 Barcelona (ES)
(72) Inventor: PRAT APARICIO, Aleix, 08036 Barcelona (ES); BRASÓ MARISTANY, Fara, 08036 Barcelona (ES); PARÉ BRUNET, Laia, 08036 Barcelona (ES)
(74) Representative: Hoffmann Eitle

(57) **Abstract**

The present invention refers to an *in vitro* method for predicting longevity in patients suffering from breast cancer, independent of age and cancer recurrence or relapse.

## Description

### FIELD OF THE INVENTION

The present invention refers to the medical field. Particularly, the present invention refers to an *in vitro* method for predicting longevity, in patients suffering from breast cancer, preferably early-stage breast cancer, independent of age and cancer recurrence or relapse.

### STATE OF THE ART

Cancer survival rates vary by the type of cancer, stage at diagnosis, treatment given and many other factors, including country.

Regarding breast cancer, there is an unmet medical need of finding a method able to identify patients suffering from breast cancer who have a longer overall survival (OS) or longevity and differentiate them from those patients suffering from breast cancer who have a shorter overall survival or longevity, regardless of whether the patient is suffering from cancer recurrence or relapse, and/or independent of patient's age.

Being able to identify those patient with longer longevity, and differentiate them from those patients with shorter longevity, regardless of whether the patient is suffering from recurrence or relapse and/or independent of patient's age, is of utmost importance to physicians. This way, once it is identified whether the patient may have a longer or shorter longevity, regardless of whether the patient is suffering from recurrence or relapse and/or independent of patient's age, physicians could adapt the intensity of treatment to be administered according to the patient's longevity and considering recurrence or relapse and/or age as secondary variables.

The present invention is focused on solving this problem and it is directed to a method for predicting longevity, in patients suffering from breast cancer, preferably early-stage breast cancer, independent of age and cancer recurrence or relapse.

### DESCRIPTION OF THE INVENTION

As explained above, the present invention refers to an *in vitro* method for predicting longevity independent of age in patients suffering from early-stage breast cancer.

The inventors of the present invention have identified the association between the level of expression of immune genes, particularly the immune genes CD27, CD79A, HLA-C, IGJ, IGKC, IGL, IGLV3-25, IL2RG, CXCL8, LAX1, NTN3, PIM2, POU2AF1 and/or TNFRSF17, and OS or longevity, independent of the patient's age, in patients with breast cancer that did not experience a relapse.

So, the "special technical feature", i.e., the technical feature that defines the contribution of the present invention over the prior art, and that confers unity to the present invention, is the use of immune genes for predicting longevity in patients suffering from breast cancer, preferably early-stage breast cancer, independent of age and cancer recurrence or relapse.

Particularly, the inventors of the present invention obtained, from two cohorts of early-stage breast cancer patients: SCAN-B (N=4470 patients without breast cancer relapse) and METABRIC (n=1133 without breast cancer relapse), gene expression and clinical data, including baseline characteristics and long-term follow-up. The association of the 14-gene IGG signature: CD27, CD79A, HLA-C, IGJ, IGKC, IGL, IGLV3-25, IL2RG, CXCL8, LAX1, NTN3, PIM2, POU2AF1 and/or TNFRSF17, with OS in the absence of recurrence or relapse was assessed in both cohorts. Additionally, the core IGG genes were identified which are strongly associated with longevity and examined their relationship with age (**Table 2** and **Table 3**). On the other hand, the performance of synergistic two-pair combinations of the genes was assayed through addition, subtraction, multiplication, and division to enhance the prediction of longevity, such as it can be seen in several two-gene combinations shown in **Table 4** and **Table 5.**

Of note, such as it is shown in the Examples below, the continuous expression of the 14-gene IGG signature was significantly associated with OS without recurrence or relapse in both cohorts (p<0.0001), independent of hormone receptor expression, HER2 status, tumor stage, and nodal status. Among the 14 IGG signature genes, the continuous expression of the genes CD27, TNFRSF17, IGJ, POU2AF1, CD79A, PIM2, IL2RG, LAX1 or IGKC was significantly associated with longevity (p<0.0001) across both datasets, defining the Core IGG genes (CIGG). Moreover, combining the expression of two genes provided more prognostic information than a single gene (p<0.001).

So, immune genes are associated with improved longevity in patients who have experienced breast cancer, suggesting a potential role in predicting prolonged survival independent of cancer recurrence or relapse and age.

Of note, the inventors of the present invention indicate that there are specific mechanisms through which these genes contribute to improved longevity beyond breast cancer recurrence or relapse and suggest that they collectively create an immune microenvironment that supports prolonged overall survival. Understanding the intricate interplay between these genes and their downstream effects on immune function is crucial for unlocking their therapeutic potential and advancing personalized treatment strategies for breast cancer patients.

Intriguingly, as explained above, combined pairs of the genes revealed that the sum of two of them provided more prognostic information than individual genes in a significant proportion of cases. This innovative approach to combining gene expressions demonstrated the potential for refining prognostic models, paving the way for more nuanced predictions of longevity in breast cancer survivors.

In conclusion, the present invention underscores the pivotal role of immune gene expression, particularly the 14-gene IGG signature and the identified Core IGG genes, in predicting extended longevity in breast cancer survivors. The exploration of novel combinations of these genes opens new avenues for refining prognosis and longevity models, ultimately contributing to the advancement of personalized treatment strategies for patients.

Consequently, the first embodiment of the present invention refers to an *in vitro* method for identifying biomarker signatures for predicting longevity in patients suffering from breast cancer, independent of age and cancer recurrence or relapse, which comprises: a) Measuring the level of expression of at least an immune gene selected from the group consisting of: CD27, CD79A, HLA-C, IGJ, IGKC, IGL, IGLV3-25, IL2RG, CXCL8, LAX1, NTN3, PIM2, POU2AF1 and/or TNFRSF17, or any combination thereof comprising between 2 and 14 of said genes, in a biological sample obtained from the patient; b) wherein if a deviation of the expression level is identified, as compared with a pre-established reference value, this is indicative that the biomarker signature may be used for predicting longevity in patients suffering from breast cancer, independent of age and cancer recurrence or relapse.

In a preferred embodiment, the present invention refers to an *in vitro* method for identifying biomarker signatures for predicting longevity in patients suffering from breast cancer, independent of age and cancer recurrence or relapse, which comprises: a) Measuring the level of expression of at least an immune gene selected from the group consisting of: CD27, TNFRSF17, IGJ, POU2AF1, CD79A, PIM2, IL2RG, LAX1 and/or IGKC, or any combination thereof comprising between 2 and 9 of said genes, in a biological sample obtained from the patient; b) wherein if a deviation of the expression level is identified, as compared with a pre-established reference value, this is indicative that the biomarker signature may be used for predicting longevity in patients suffering from breast cancer, independent of age and cancer recurrence or relapse.

In a preferred embodiment, the present invention refers to an *in vitro* method for identifying biomarker signatures for predicting longevity in patients suffering from breast cancer, independent of age and cancer recurrence or relapse, which comprises: a) Measuring the level of expression of at least two immune genes selected from the group consisting of: CD27, TNFRSF17, IGJ, POU2AF1, CD79A, PIM2, IL2RG, LAX1 and/or IGKC in a biological sample obtained from the patient; b) determining a combination score value by calculating the ratio of the expression of the 2 genes; and c) wherein if a deviation of the combination score value is identified, as compared with a pre-established reference value, this is indicative that the biomarker signature may be used for predicting longevity in patients suffering from breast cancer, independent of age and cancer recurrence or relapse.

In a preferred embodiment, the present invention refers to an *in vitro* method for predicting longevity in patients suffering from breast cancer, independent of age and cancer recurrence or relapse, which comprises: a) Measuring the level of expression of at least an immune gene selected from the group consisting of: CD27, TNFRSF17, IGJ, POU2AF1, CD79A, PIM2, IL2RG, LAX1 and/or IGKC, or any combination thereof comprising between 2 and 9 of said genes, in a biological sample obtained from the patient; b) wherein the identification of an increased level of expression, as compared with a pre-established reference value, is indicative that the patient suffering from breast cancer has a longer longevity independent of age and cancer recurrence or relapse.

In a preferred embodiment, the present invention refers to an *in vitro* method for predicting longevity in patients suffering from breast cancer, independent of age and cancer recurrence or relapse, which comprises: a) Measuring the level of expression of at least two immune genes selected from the group consisting of: CD27, TNFRSF17, IGJ, POU2AF1, CD79A, PIM2, IL2RG, LAX1 and/or IGKC, in a biological sample obtained from the patient; b) determining a combination score value by calculating the ratio of the expression of the 2 genes; and c) wherein if an increase of the combination score value is identified, as compared with a pre-established reference value, this is indicative that the patient suffering from breast cancer has a longer longevity independent of age and cancer recurrence or relapse.

In a preferred embodiment, the present invention refers to an *in vitro* method for predicting longevity, in patients suffering from breast cancer, independent of age and cancer recurrence or relapse, which comprises: a) Measuring the level of expression of at least one of the two-immune genes combinations of **Table 4** or **Table 5** in a biological sample obtained from the patient; b) determining a combination score value by calculating the ratio of the expression of the 2 genes; and c) wherein if an increase of the combination score value is identified, as compared with a pre-established reference value, this is indicative that the patient suffering from breast cancer has a longer longevity independent of age and cancer recurrence or relapse.

The second embodiment of the present invention refers to the *vitro* use at least one immune gene selected from the group consisting of: CD27, CD79A, HLA-C, IGJ, IGKC, IGL, IGLV3-25, IL2RG, CXCL8, LAX1, NTN3, PIM2, POU2AF1 and/or TNFRSF17, or any combination thereof comprising between 2 and 14 of said genes, for identifying biomarker signatures for predicting longevity, in patients suffering from breast cancer, independent of age and cancer recurrence or relapse, or for predicting longevity in patients suffering from breast cancer, independent of age and cancer recurrence or relapse.

In a preferred embodiment the present invention refers to the *in vitro* at least one immune gene selected from the group consisting of: CD7, TNFRSF17, IGJ, POU2AF1, CD79A, PIM2, IL2RG, LAX1 and/or IGKC, or any combination thereof comprising between 2 and 9 of said genes, for identifying biomarker signatures for predicting longevity, in patients suffering from breast cancer, independent of age and cancer recurrence or relapse, or for predicting longevity in patients suffering from breast cancer, independent of age and cancer recurrence or relapse.

In a preferred embodiment the present invention refers to the *in vitro* use at least two immune genes selected from the group consisting of: CD27, TNFRSF17, IGJ, POU2AF1, CD79A, PIM2, IL2RG, LAX1 and/or IGKC, for identifying biomarker signatures predicting longevity in patients suffering from breast cancer, independent of age and cancer recurrence or relapse, or for predicting longevity in patients suffering from breast cancer, independent of age and cancer recurrence or relapse.

In a preferred embodiment the present invention refers to the *in vitro* use of at least one of the two-immune genes combinations of **Table 4** or **Table 5** for predicting longevity in patients suffering from breast cancer, independent of age and cancer recurrence or relapse.

The third embodiment of the present invention refers to the *in vitro* use of a kit comprising reagents for measuring the level of expression of at least one immune gene selected from the group consisting of: CD27, TNFRSF17, IGJ, POU2AF1, CD79A, PIM2, IL2RG, LAX1 and/or IGKC, or any combination thereof comprising between 2 and 9 of said genes, for predicting longevity in patients suffering from breast cancer, independent of age and cancer recurrence or relapse.

In a preferred embodiment the present invention refers to the *in vitro* use of a kit comprising reagents for measuring the level of expression of at least two immune gene selected from the group consisting of: CD27, TNFRSF17, IGJ, POU2AF1, CD79A, PIM2, IL2RG, LAX1 and/or IGKC, for predicting longevity in patients suffering from breast cancer, independent of age and cancer recurrence or relapse.

In a preferred embodiment the present invention refers to the *in vitro* use of a kit comprising reagents for measuring the level of expression of at least one of the two-immune genes combinations of **Table 4** or **Table 5** or for predicting longevity in patients suffering from breast cancer, independent of age and cancer recurrence or relapse.

In a preferred embodiment, the sample is selected from: tissue, blood, serum or plasma.

In a preferred embodiment, the patient is suffering from early-stage breast cancer.

In a preferred embodiment, the patient is suffering from hormone receptor-positive and HER2-negative (HR+/HER2-) or human epidermal growth factor receptor 2-positive (HER2+) or triple-negative breast cancer.

In a preferred embodiment the present invention can be used for identifying those patients suffering from breast cancer with longer longevity and differentiate them from those patients suffering from breast cancer with shorter longevity.

In a preferred embodiment the present invention can be used for selecting the therapy to be administered to the patient suffering from breast cancer, once the longevity of the patient has been predicted by carrying out the present invention. Thus, the strength or intensity of the therapy to be administered to the patient is selected according to the longevity assessed by means of the present invention. If immune genes are overexpressed, this opens the possibility of less intensive treatment even in older patients. If immune genes are downregulated more intensive treatment should be followed even in young patients. The treatments to be administered, according to the assessed longevity, can be summarized as follows:
- Surgery.
- Radiotherapy.
- Systemic therapy:
   ∘ Chemotherapy.
   ∘ Anti-HER2 therapy.
   ∘ Endocrine therapy.
   ∘ Immunotherapy.
- Follow-up care: Care after primary breast cancer treatment, otherwise called 'follow-up care', can be intensive involving regular laboratory tests in asymptomatic people to try to achieve earlier detection of possible metastases. A review has found that follow-up programs involving regular physical examinations and yearly mammography alone are as effective as more intensive programs consisting of laboratory tests in terms of early detection of recurrence or relapse, overall survival and quality of life.

In a preferred embodiment, the present invention is a computer-implemented invention, wherein a processing unit (hardware) and a software are configured to: a) Receive the expression level values of any of the above cited biomarkers or signatures, b) process the expression level values received for finding substantial variations or deviations, and c) provide an output through a terminal display of the variation or deviation of the expression level.

In a preferred embodiment, the method of the invention further comprises determining or measuring tumor stage and/or nodal status, using image techniques, for instance by CT scan, ultrasound and/or mammography.

For the purpose of the present invention the following terms are defined:
- The term "pre-established reference value", when referring to the level of expression of the biomarkers described in the present invention, refers to level measured in control subjects or considering the geometric mean level of house-keeping genes. A "reference" value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value must be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data.
- The term "variation or deviation" refers to a value which is above or below the pre-established reference value.
- By the term "comprising" is meant the inclusion, without limitation, of whatever follows the word "comprising". Thus, use of the term "comprising" indicates that the listed elements are required or mandatory, but that other elements are optional and may or may not be present.
- By "consisting of" is meant the inclusion, with limitation to whatever follows the phrase "consisting of. Thus, the phrase "consisting of" indicates that the listed elements are required or mandatory, and that no other elements may be present.

### Brief description of the figures

**Figure 1****.** Assessment of the 14-gene IGG signature for predicting longevity in patients with breast cancer without documented recurrence or relapse.
**Figure 2****.** Association of IGG signature with OS without recurrence or relapse in (a) n=1133 samples of METABRIC and **(b)** n= 4470 samples of SCAN-B.
**Figure 3****.** Correlation between IGG signature and age, and distribution of IGG groups (low, medium, high) across age groups.
**Figure 4****.** Association of IGG and OS without recurrence across age groups.
**Figure 5****.** IGG expression in HR+/HER2-, HER2+ and TNBC, and distribution of IGG groups (low, medium, high) across age groups and breast cancer subtype.
**Figure 6****.** Genes associated with better OS without recurrence or relapse in univariate Cox regression models in METABRIC and SCAN-B.
**Figure 7****.** Expression of 7 CIGGs and IGG signatures in the METABRIC dataset.
**Figure 8****.** Correlation between 7 CIGG genes and age in the METABRIC dataset.
**Figure 9****.** Amount of variation explained in prognosis defined by chi-square (χ2) statistics from likelihood ratio tests.
**Figure 10****.** Association of the combination of **(a)** 2 CIGG genes and **(b)** a single gene with OS without recurrence or relapse in METABRIC and SCAN-B combined.

### Detailed description of the invention

The present invention is illustrated by means of the examples set below, without the intention of limiting its scope of protection.

### Example 1. MATERIAL AND METHODS

### Example 1.1. Patient Datasets

The METABRIC dataset was sourced from the cBio Cancer Genomics Portal (http://cbioportal.org), while data from the most recent version of the SCAN-B dataset were obtained from Mendeley Data (https://data.mendeley.com/datasets/yzxtxn4nmd). Patients who experienced breast cancer recurrence or relapse were systematically excluded from both datasets. The final analysis comprised 4,470 patients from SCAN-B and 1,133 patients from METABRIC, ensuring a cohort that did not encounter recurrence or relapse during the follow-up period. The median follow-up in these patients was 96.9 months in SCAN-B, and 185.0 months in METABRIC.

### Example 1.2. Gene Expression

Z-scores or log2-transformed normalized expression values were employed for single genes and for deriving the 14-gene IGG signature, depending on the available gene expression data for each dataset. The IGG signature, previously identified through unsupervised clustering of 550 node-negative breast tumors, was externally validated across diverse clinical cohorts involving over 1,000 patients. Comprising 14 genes associated with various aspects of lymphocyte progenitor maturation, activation, differentiation, immunoglobulin production, chemotaxis, and lymphocyte activity regulation, the IGG score was calculated as the mean expression of the available genes in datasets where the expression of some IGG genes was missing.

### Example 1.3. Core IGG Genes (CIGGs)

Genes consistently associated with longevity across both datasets were identified as Core IGG genes (CIGGs). We further investigated whether the combined assessment of pairs of CIGGs enhanced longevity prediction compared to individual genes. Various mathematical operations, including addition, subtraction, multiplication, and ratio, were employed to combine the expressions of two CIGGs.

### Example 1.4. Statistical Analysis

Univariate and multivariable Cox regression models were employed, with the goodness-of-fit of each model assessed through the chi-square (χ2) statistic obtained from a likelihood ratio test. In addition, correlations between two variables were evaluated using the Pearson method. The comparison between models combining two CIGGs and models utilizing individual genes aimed to ascertain the optimal approach for enhancing longevity prediction. A 2-sided alpha of 0.05 was set as the significance level for all statistical analyses.

### Example 1. RESULTS

### Example 1.1. IGG association with longevity

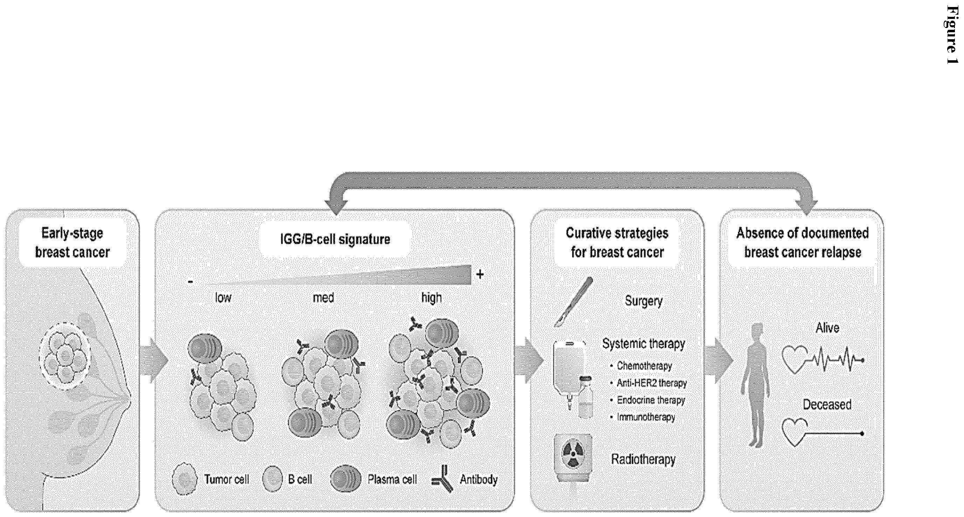

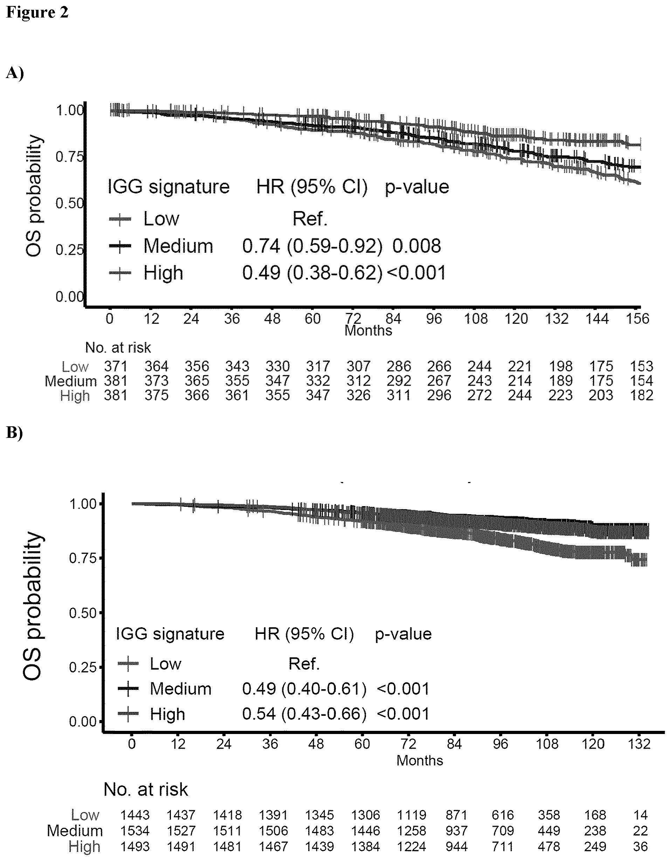

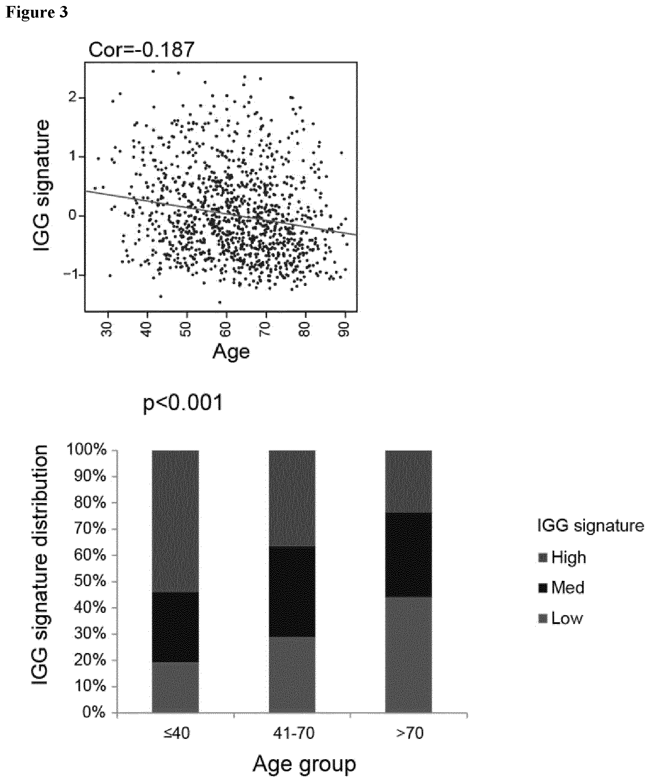

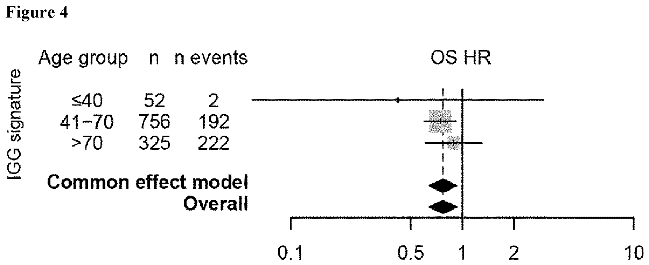

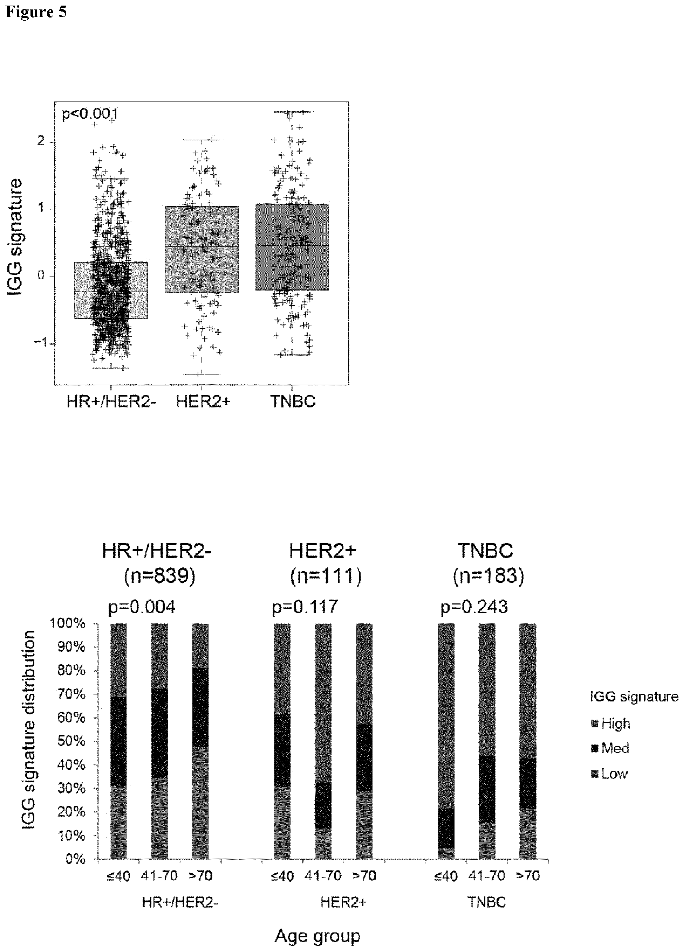

Our study addresses this gap by exploring the association between the 14-gene IGG signature and OS without recurrence or relapse, defined as longevity. Leveraging comprehensive gene expression and clinical data from two distinct cohorts of early-stage breast cancer patients, namely SCAN-B and METABRIC (**Figure 1**). The continuous expression of the 14-gene IGG signature was significantly associated with OS without recurrence or relapse in both cohorts (p<0.0001), independent of hormone receptor expression, HER2 status, tumor size, and nodal status (**Table 1** and **Figure 2**). A moderate correlation between the IGG signature and age was observed (**Figure 3**). In patients aged below ≤70 years old, IGG signature was significantly associated with OS without relapse recurrence or relapse independently of age (METABRIC: HR [95%CI]=0.82 [0.66-1.00], p=0.050; SCAN-B: HR [95%CI]=0.82 [0.71-0.94], p=0.005). Across age groups, a statistically significant association of the IGG signature with OS was observed in patients aged >40 and ≤70 years old (**Figure 4**). IGG signature was higher in HER2+ and TNBC tumors. Additionally, IGG-low disease increased with age, especially in patients aged >70 years old and with HR+/HER2- breast cancer (**Figure 5**). Although IGG expression is independent of age and tumor subtype, the three variables are related, with older ages associated with lower IGG tumor infiltration.

**Table 1. Association of IGG signature with OS without recurrence or relapse in Cox regression models.**

| **Study** | | **n** | **OS events** | **HR (95%CI)** | **p-value** |
|---|---|---|---|---|---|
| METABRIC | univariate | 1133 | 416 | 0.67 (0.58-0.77) | <0.0001 |
| | bivariate* | 1133 | 416 | 0.72 (0.62-0.84) | <0.0001 |
| | multivariable** | 823 | 287 | 0.73 (0.60-0.88) | 0.0012 |
| SCAN-B | univariate | 4470 | 508 | 0.78 (0.73-0.85) | <0.001 |
| | bivariate* | 4361 | 499 | 0.77 (0.72-0.84) | <0.001 |
| | multivariable** | 4211 | 477 | 0.79 (0.73-0.86) | <0.001 |

| | | | | | |
|---|---|---|---|---|---|
| *adjusted by IHC subtype; **adjusted by IHC subtype, T stage and N status | | | | | |

### Example 1.2. Identification of the CIGGs

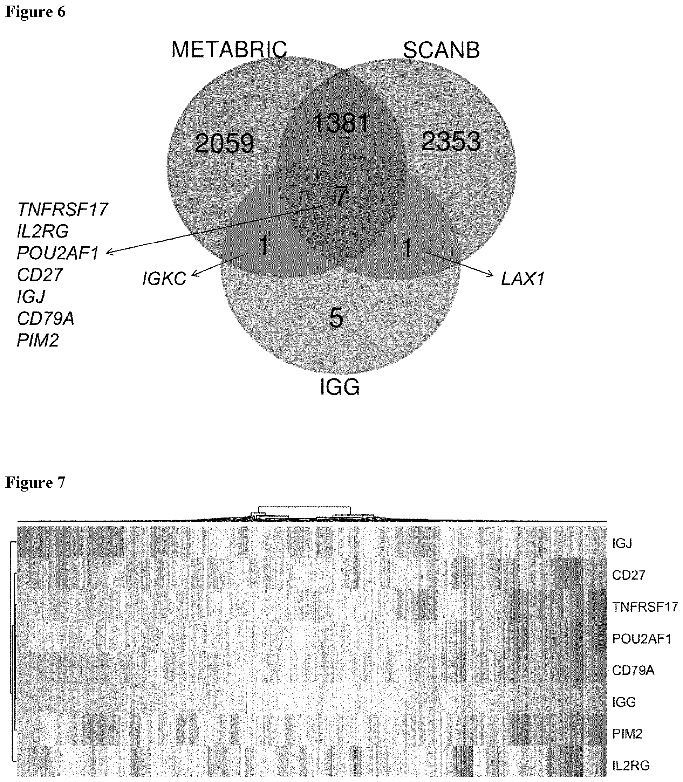

To identify the key genes influencing longevity within the IGG signature, we assessed the association of the genes composing the signature with OS without recurrence or relapse in the METABRIC and SCAN-B datasets. Seven genes (*TNFRSF17* [BCMA], *IL2RG, POU2AF1, CD27, IGJ* [JCHAIN], *CD79A,* and *PIM2*) across both datasets exhibited a statistically significant association with longevity (p<0.0001) defining the core IGG genes (CIGG) (**Figure 6** and **Table 2**). A moderate correlation coefficient of 0.63 was observed among CIGGs (**Figure 7**). Additionally, IGKC and LAX1 were also significantly associated with longevity (p<0.0001) in METABRIC and SCAN-B datasets, respectively (**Figure 6** and **Table 2**).

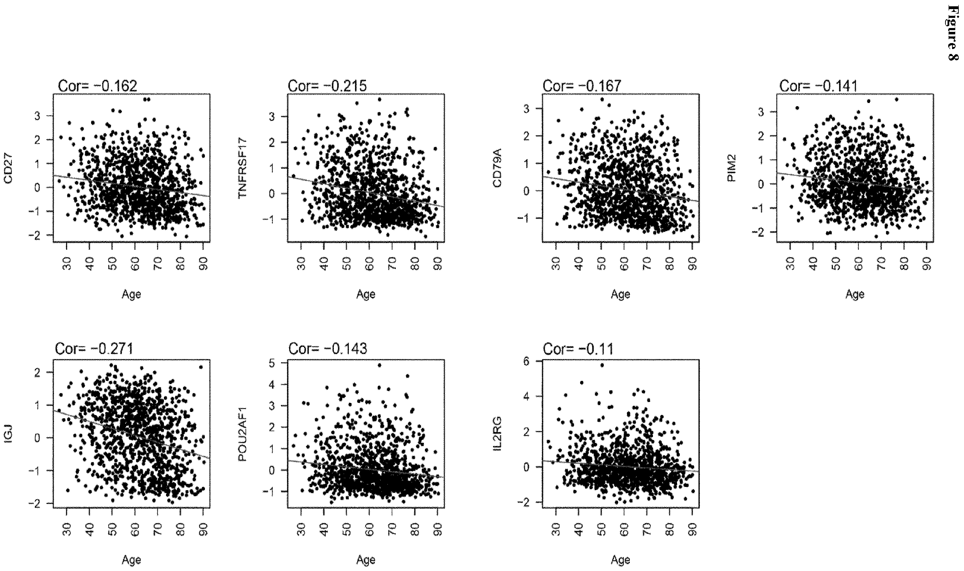

No substantial association was observed between the expression of each CIGG and age (**Figure 8**). In patients with less than 70 years of age, the continuous expression of the 7 CIGGs were significantly associated with longevity in both cohorts (p<0.0001), independent of age (as a continuous variable), hormone receptor expression, HER2 status, tumor size, and nodal status (**Table 3**).

**Table 2. Association of individual genes with OS without recurrence or relapse in univariate Cox regression models.**

| **Study** | **gene** | **n** | **OS events** | **HR (95%CI)** | **pvalue** |
|---|---|---|---|---|---|
| METABRIC | CD27 | 1133 | 416 | 0.76 (0.68-0.84) | <0.0001 |
| | TNFRSF17 | 1133 | 416 | 0.70 (0.62-0.78) | <0.0001 |
| | IGJ | 1133 | 416 | 0.69 (0.62-0.76) | <0.0001 |
| | POU2AF1 | 1133 | 416 | 0.78 (0.7-0.88) | <0.0001 |
| | CD79A | 1133 | 416 | 0.75 (0.67-0.84) | <0.0001 |
| | PIM2 | 1133 | 416 | 0.80 (0.72-0.89) | <0.0001 |
| | IL2RG | 1133 | 416 | 0.83 (0.75-0.92) | 0.0005 |
| | IGKC | 1133 | 416 | 0.78 (0.71-0.85) | <0.0001 |
| SCAN-B | CD27 | 4470 | 508 | 0.73 (0.66-0.80) | <0.0001 |
| | TNFRSF17 | 4470 | 508 | 0.73 (0.66-0.80) | <0.0001 |
| | IGJ | 4470 | 508 | 0.68 (0.63-0.74) | <0.0001 |
| | POU2AF1 | 4470 | 508 | 0.74 (0.67-0.82) | <0.0001 |
| | CD79A | 4470 | 508 | 0.75 (0.68-0.82) | <0.0001 |
| | PIM2 | 4470 | 508 | 0.84 (0.76-0.93) | 0.0004 |
| | IL2RG | 4470 | 508 | 0.80 (0.73-0.87) | <0.0001 |
| | LAX1 | 4470 | 508 | 0.82 (0.75-0.91) | 0.0001 |

**Table 3. Association of each CIGG gene with OS without recurrence or relapse in patients with less than 70 years of age in a multivariable* Cox regression model.**

| **Study** | **gene** | **n** | **OS events** | **HR (95%CI)** | **pvalue** |
|---|---|---|---|---|---|
| METABRIC + SCAN-B | *CD27* | 3686 | 290 | 0.82 (0.73-0.93) | 0.0013 |
| | *TNFRSF17* | 3686 | 290 | 0.85 (0.74-0.96) | 0.0111 |
| | *IGJ* | 3686 | 290 | 0.90 (0.84-0.97) | 0.0046 |
| | *POU2AF1* | 3686 | 290 | 0.83 (0.73-0.95) | 0.0064 |
| | *CD79A* | 3686 | 290 | 0.84 (0.76-0.94) | 0.0013 |
| | *PIM2* | 3686 | 290 | 0.86 (0.75-0.99) | 0.0315 |
| | *IL2RG* | 3686 | 290 | 0.86 (0.76-0.96) | 0.0096 |

| | | | | | |
|---|---|---|---|---|---|
| *adjusted by study, age (as a continuous variable), IHC subtype, T stage and N status | | | | | |

### Example 1.3. Combination of CIGGs and longevity

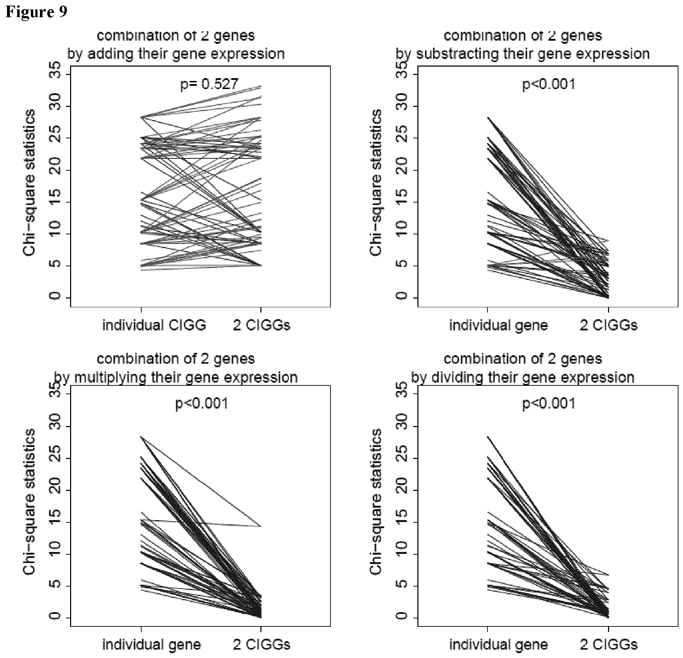

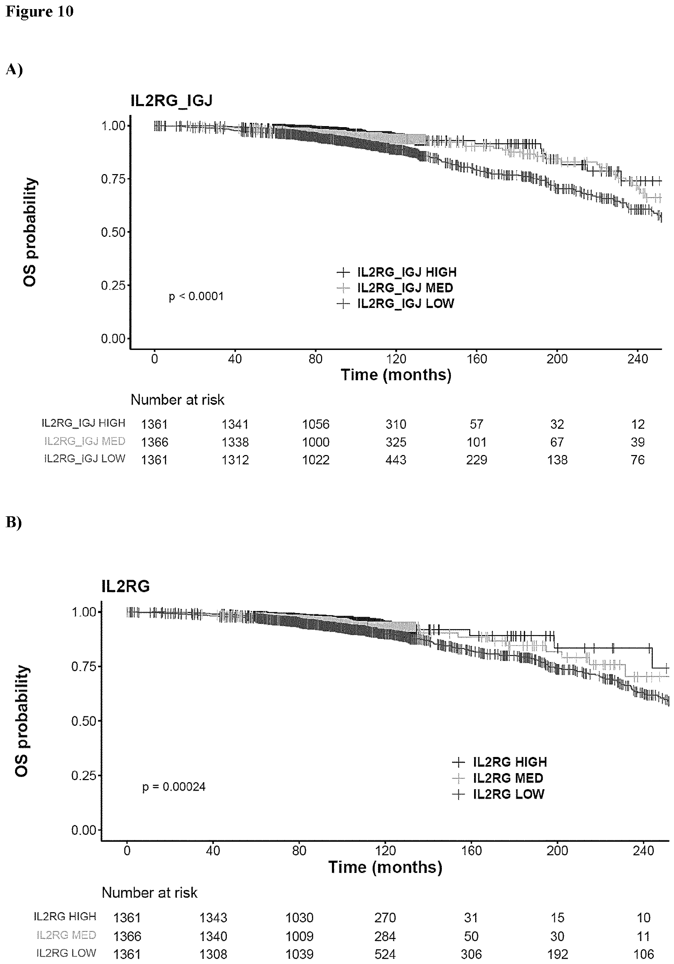

Combining the expression of two CIGGs by adding their gene expression provided more prognostic information than a single gene (p<0.001) in 16 combinations (**Table 4** and **Figures 9-10**) in patients <70 years old. Two-gene subtraction, multiplication, or ratio decreased the prognostic information (**Table 4 and** **Figure 9**). All the combinations of 2 CIGGs in METABRIC and SCAN-B combined were significantly associated with low risk of death, independently of study and treatment arm (**Table 5**).

**Table 4. Amount of variation explained in prognosis defined by chi-square (χ2) statistics from likelihood ratio tests.**

| **Study** | **Gene combination** | **Gene1** | **Gene2** | **Sum** | **Substract** | **Multiply** | **Ratio** |
|---|---|---|---|---|---|---|---|
| **METAB RIC + SCANB** | ***TNFRSF17_IL2RG*** | 25.142 | 15.355 | 25.446 | 3.056 | 3.136 | 2.794 |
| | ***TNFRSF17_CD27*** | 25.142 | 24.177 | 27.851 | 0.03 | 0.805 | 0.072 |
| | ***TNFRSF17_IGJ*** | 25.142 | 28.325 | 30.338 | 3.609 | 0.317 | 0.585 |
| | ***TNFRSF17_CD79A*** | 25.142 | 23.424 | 26.32 | 0.101 | 0.921 | 0.001 |
| | ***IL2RG_IGJ*** | 15.355 | 28.325 | 32.906 | 5.808 | 14.279 | 0.022 |
| | ***POU2AF1_CD27*** | 21.892 | 24.177 | 25.157 | 0.273 | 1.322 | 4.374 |
| | ***POU2AF1_IGJ*** | 21.892 | 28.325 | 31.327 | 2.641 | 0.661 | 0.05 |
| | ***POU2AF1_CD79A*** | 21.892 | 23.424 | 23.701 | 0.779 | 1.491 | 1.069 |
| | ***CD27_IGJ*** | 24.177 | 28.325 | 33.231 | 1.676 | 2.592 | 0.019 |
| | ***CD27_CD79A*** | 24.177 | 23.424 | 25.342 | 0.016 | 1.171 | 0.048 |
| | ***IGJ_CD79A*** | 28.325 | 23.424 | 31.566 | 1.727 | 3.113 | 0.01 |
| **METAB RIC** | ***IL2RG_IGKC*** | 4.830 | 8.528 | 9.524 | 0.435 | 2.091 | 0.965 |
| | ***POU2AF1_IGKC*** | 10.166 | 8.528 | 10.942 | 0.000 | 0.009 | 4.560 |
| | ***CD27_IGKC*** | 10.405 | 8.528 | 11.254 | 0.032 | 1.545 | 0.073 |
| | ***PIM2_IGKC*** | 5.923 | 8.528 | 8.731 | 0.490 | 0.104 | 1.505 |
| **SCANB** | ***PIM2_LAX1*** | 4.368 | 5.081 | 5.108 | 0.054 | 0.138 | 1.035 |

**Table 5. Association of 2 CIGGs (in combination into a single score) with OS without recurrence or relapse in patients <70 years old in METABRIC and SCAN-B datasets.**

| **Study** | **Gene combination** | **n** | **n_event** | **HR Low** | **95%CI High** | **95%CI** | **pvalue** |
|---|---|---|---|---|---|---|---|
| **METABRIC + SCANB** | ***TNFRSF17_IL2RG*** | 4088 | 350 | 0.839 | 0.781 | 0.901 | 1.36E-06 |
| | ***TNFRSF17_CD27*** | 4088 | 350 | 0.853 | 0.802 | 0.907 | 3.67E-07 |
| | ***TNFRSF17_IGJ*** | 4088 | 350 | 0.862 | 0.817 | 0.909 | 5.50E-08 |
| | ***TNFRSF17_CD79A*** | 4088 | 350 | 0.861 | 0.811 | 0.914 | 8.40E-07 |
| | ***IL2RG_IGJ*** | 4088 | 350 | 0.832 | 0.781 | 0.886 | 9.75E-09 |
| | ***POU2AF1_CD27*** | 4088 | 350 | 0.862 | 0.811 | 0.916 | 1.69E-06 |
| | ***POU2AF1_IGJ*** | 4088 | 350 | 0.855 | 0.809 | 0.904 | 3.43E-08 |
| | ***POU2AF1_CD79A*** | 4088 | 350 | 0.870 | 0.820 | 0.923 | 3.59E-06 |
| | ***CD27_IGJ*** | 4088 | 350 | 0.853 | 0.808 | 0.900 | 8.61E-09 |
| | ***CD27_CD79A*** | 4088 | 350 | 0.868 | 0.819 | 0.919 | 1.15E-06 |
| | ***IGJ_CD79A*** | 4088 | 350 | 0.860 | 0.816 | 0.907 | 2.35E-08 |
| **METABRIC** | ***IL2RG*_*IGKC*** | 808 | 194 | 0.877 | 0.805 | 0.955 | 2.54E-03 |
| | ***POU2AF1_IGKC*** | 808 | 194 | 0.875 | 0.807 | 0.948 | 1.11E-03 |
| | ***CD27_IGKC*** | 808 | 194 | 0.875 | 0.809 | 0.946 | 7.70E-04 |
| | ***PIM2_IGKC*** | 808 | 194 | 0.889 | 0.821 | 0.962 | 3.49E-03 |
| **SCANB** | ***PIM2_LAX1*** | 3280 | 156 | 0.916 | 0.855 | 0.982 | 1.29E-02 |

## Claims

1. *In vitro* method for identifying biomarker signatures for predicting longevity, in patients suffering from breast cancer, independent of age and cancer recurrence or relapse, which comprises:
a. Measuring the level of expression of at least an immune gene selected from the group consisting of: CD27, CD79A, HLA-C, IGJ, IGKC, IGL, IGLV3-25, IL2RG, CXCL8, LAX1, NTN3, PIM2, POU2AF1 and/or TNFRSF17, or any combination thereof, in a biological sample obtained from the patient;
b. Wherein if a deviation of the expression level is identified, as compared with a pre-established reference value, this is indicative that the biomarker signature may be used for predicting longevity, in patients suffering from breast cancer, independent of age and cancer recurrence or relapse.

2. *In vitro* method for identifying biomarker signatures for predicting longevity, in patients suffering from breast cancer, independent of age and cancer recurrence or relapse, according to claim 1, which comprises:
a. Measuring the level of expression of at least an immune gene selected from the group consisting of: CD27, TNFRSF17, IGJ, POU2AF1, CD79A, PIM2, IL2RG, LAX1 and/or IGKC, or any combination thereof, in a biological sample obtained from the patient;
b. Wherein if a deviation of the expression level is identified, as compared with a pre-established reference value, this is indicative that the biomarker signature may be used for predicting longevity, in patients suffering from breast cancer, independent of age and cancer recurrence or relapse.

3. *In vitro* method for identifying biomarker signatures for predicting longevity independent, in patients suffering from breast cancer, independent of age and cancer recurrence or relapse, according to any of the previous claims, which comprises:
a. Measuring the level of expression of at least two immune genes selected from the group consisting of: CD27, TNFRSF17, IGJ, POU2AF1, CD79A, PIM2, IL2RG, LAX1 and/or IGKC, in a biological sample obtained from the patient;
b. Determining a combination score value by calculating the ratio of the expression of the 2 genes; and
c. Wherein if a deviation of the combination score value is identified, as compared with a pre-established reference value, this is indicative that the biomarker signature may be used for predicting longevity, in patients suffering from breast cancer, independent of age and cancer recurrence or relapse.

4. *In vitro* method for predicting longevity, in patients suffering from breast cancer, independent of age and cancer recurrence or relapse, according to any of the previous claims, which comprises:
a. Measuring the level of expression of at least an immune gene selected from the group consisting of: CD27, TNFRSF17, IGJ, POU2AF1, CD79A, PIM2, IL2RG, LAX1 and/or IGKC, or any combination thereof, in a biological sample obtained from the patient;
b. Wherein the identification of an increased level of expression, as compared with a pre-established reference value, is indicative that the patient suffering from breast cancer has a longer longevity independent of age and cancer recurrence or relapse.

5. *In vitro* method for predicting longevity, in patients suffering from breast cancer, independent of age and cancer recurrence or relapse, according to any of the previous claims, which comprises:
a. Measuring the level of expression of at least two immune genes selected from the group consisting of: CD27, TNFRSF17, IGJ, POU2AF1, CD79A, PIM2, IL2RG, LAX1 and/or IGKC, in a biological sample obtained from the patient;
b. Determining a combination score value by calculating the ratio of the expression of the 2 genes; and
c. Wherein if an increase of the combination score value is identified, as compared with a pre-established reference value, this is indicative that the patient suffering from breast cancer has a longer longevity independent of age and cancer recurrence or relapse.

6. *In vitro* method for predicting longevity, in patients suffering from breast cancer, independent of age and cancer recurrence or relapse, according to any of the previous claims, which comprises:
a. Measuring the level of expression of at least one of the two-immune genes combinations of **Table 4** or **Table 5** in a biological sample obtained from the patient;
b. Determining a combination score value by calculating the ratio of the expression of the 2 genes; and
c. Wherein if an increase of the combination score value is identified, as compared with a pre-established reference value, this is indicative that the patient suffering from breast cancer has a longer longevity independent of age and cancer recurrence or relapse.

7. *In vitro* method, according to any of the previous claims, wherein the sample is selected form: tissue, blood, serum or plasma.

8. *In vitro* method, according to any of the previous claims, wherein the patient is suffering from hormone receptor positive breast cancer and HER2-negative (HR+/HER2-), human epidermal growth factor receptor 2-positive (HER2+) or triple-negative breast cancer.

9. *In vitro* use at least one immune gene selected from the group consisting of: CD27, CD79A, HLA-C, IGJ, IGKC, IGL, IGLV3-25, IL2RG, CXCL8, LAX1, NTN3, PIM2, POU2AF1 and/or TNFRSF17, or any combination thereof, for identifying biomarker signatures for predicting longevity in patients suffering from breast cancer, independent of age and cancer recurrence or relapse, or for predicting longevity in patients suffering from breast cancer, independent of age and cancer recurrence or relapse.

10. *In vitro* use at least one immune gene selected from the group consisting of: CD27, TNFRSF17, IGJ, POU2AF1, CD79A, PIM2, IL2RG, LAX1 and/or IGKC, or any combination thereof, according to claim 9, for identifying biomarker signatures for predicting longevity in patients suffering from breast cancer, independent of age and cancer recurrence or relapse, or for predicting longevity in patients suffering from breast cancer, independent of age and cancer recurrence or relapse.

11. *In vitro* use at least two immune genes selected from the group consisting of: CD27, TNFRSF17, IGJ, POU2AF1, CD79A, PIM2, IL2RG, LAX1 and/or IGKC, according to claims 9 or 10, for identifying biomarker signatures for predicting longevity in patients suffering from breast cancer, independent of age and cancer recurrence or relapse, or for predicting longevity in patients suffering from breast cancer, independent of age and cancer recurrence or relapse.

12. *In vitro* use at of at least one of the two-immune genes combinations of **Table 4** or **Table 5,** according to any of the claims 9 to 11, for predicting longevity in patients suffering from breast cancer, independent of age and cancer recurrence or relapse.

13. *In vitro* use of a kit comprising reagents for measuring the level of expression of at least one immune gene selected from the group consisting of: CD27, TNFRSF17, IGJ, POU2AF1, CD79A, PIM2, IL2RG, LAX1 and/or IGKC, or any combination thereof, for predicting longevity, in patients suffering from breast cancer, independent of age and cancer recurrence or relapse.

14. *In vitro* use of a kit comprising reagents for measuring the level of expression of at least two immune gene selected from the group consisting of: CD27, TNFRSF17, IGJ, POU2AF1, CD79A, PIM2, IL2RG, LAX1 and/or IGKC, according to claim 13, for predicting longevity, in patients suffering from breast cancer, independent of age and cancer recurrence or relapse.

15. *In vitro* use of a kit comprising reagents for measuring the level of expression of at least one of the two-immune genes combinations of **Table 4** or **Table 5,** according to any of the claims 13 or 14, for predicting longevity in patients suffering from breast cancer, independent of age and cancer recurrence or relapse.
